(19)

Europäisches
Patentamt
European
Patent Office
Office européen
des brevets

(11) **EP 4 437 973 A1**

(12) **EUROPEAN PATENT APPLICATION**
published in accordance with Art. 153(4) EPC

(43) Date of publication:
**02.10.2024 Bulletin 2024/40**

(21) Application number: **22897796.3**

(22) Date of filing: **22.11.2022**

(51) International Patent Classification (IPC):
***A61B 17/00*** *(2006.01)*

(52) Cooperative Patent Classification (CPC):
**A61B 17/00; A61B 17/12; C21D 9/00; C21D 9/52**

(86) International application number:
**PCT/CN2022/133472**

(87) International publication number:
**WO 2023/093713 (01.06.2023 Gazette 2023/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.11.2021 CN 202111425504**

(71) Applicant: **Lifetech Scientific (Shenzhen) Co., Ltd.
Shenzhen, Guangdong 518063 (CN)**

(72) Inventors:
• **CHEN, Jun
Shenzhen, Guangdong 518063 (CN)**
• **CHEN, Xianmiao
Shenzhen, Guangdong 518063 (CN)**

(74) Representative: **Michalski Hüttermann & Partner
Patentanwälte mbB
Kaistraße 16A
40221 Düsseldorf (DE)**

(54) **PUSH CABLE, PUSH SYSTEM, HEAT TREATMENT METHOD, AND METHOD FOR PREPARING PUSH CABLE**

(57) A push cable (10), a push system (100), a heat treatment method, and a push cable preparation method are provided. The push cable (10) includes a first cable body (11) and a second cable body (12) connected to one end of the first cable body (11). The first cable body (11) includes one or more first metal wires. The second cable body (12) includes one or more second metal wires. The first metal wire and the second metal wire are each made of a shape memory alloy material. The elastic energy storage efficiency of the first metal wire is less than the elastic energy storage efficiency of the second metal wire. In the environment of a human body temperature, when the first cable body (11) is experiencing bending deformation from an initial form, the first cable body cannot be restored to the initial form without the assistance of an external force. The push cable reduces the probability of the first cable body (11) flicking other parts of the heart, thereby reducing the probability of damage to heart tissue.

FIG. 1

EP 4 437 973 A1

## Description

## Technical Field

The present disclosure relates to the field of interventional medical instruments, and more particularly, to a push cable, a push system, a heat treatment method, and a push cable preparation method.

## Background Art

This section provides only background information relevant to the present disclosure and is not necessarily prior art.

With the continuous development of interventional medical instruments, transcatheter interventional minimally invasive treatment has become an important method to treat congenital heart diseases such as atrial septal defect (ASD), ventricular septal defect (VSD), patent ductus arteriosus (PDA), and patent foramen ovale (PFO).

In transcatheter interventional treatment of congenital heart defect diseases, a distal end of a push cable is detachably connected to an occluder. Then, the occluder is delivered to a heart defect site through a catheter by using the push cable. Next, the occluder is released to occlude the defect site. Finally, the push cable is disconnected from the occluder, and the push cable is withdrawn.

Existing push cables are generally stainless-steel spring tubes (such as 304 stainless steel tubes or 316L stainless steel tubes) or nickel-titanium cables. The two push cables can better comply with a bent blood vessel path and smoothly reach the defect site, and are thus widely used.

However, the push cables still have some shortcomings. Taking transcatheter atrial septal defect occlusion as an example, the distal end of the push cable is bent after the occluder occludes the defect and before the push cable is disconnected from the occluder. When the distal end of the push cable and the occluder are disconnected, the distal end of the push cable will be restored to an initial state. In this process, the push cable may flick other parts of the heart, thereby causing damage to heart tissue.

## Summary of the Disclosure

Based on this, it is necessary to provide a push cable, a push system, a heat treatment method, and a push cable preparation method, so as to reduce the probability of damage to heart tissue after the push cable is disconnected from an occluder.

A push cable includes a first cable body and a second cable body. A proximal end of the first cable body is connected to a distal end of the second cable body. The first cable body includes one or more first metal wires, and the second cable body includes one or more second metal wires. The first metal wire and the second metal wire are each made of a shape memory alloy material. The elastic energy storage efficiency of the first metal wire is less than the elastic energy storage efficiency of the second metal wire. In the environment of a human body temperature, when the first cable body is experiencing bending deformation from an initial form, the first cable body is not restored to the initial form without the assistance of an external force.

In one embodiment, the ratio of the elastic energy storage efficiency of the first metal wire to the elastic energy storage efficiency of the second metal wire is in a range of 1:1.5 to 1:5. The length ratio of the first cable body to the second cable body is in a range of 1:2 to 1:20.

In one embodiment, the push cable further includes a third cable body made of a shape memory alloy material. A distal end of the first cable body is connected to a proximal end of the third cable body.

In one embodiment, the third cable body includes one or more third metal wires. The elastic energy storage efficiency of the third metal wire is less than the elastic energy storage efficiency of the second metal wire and greater than the elastic energy storage efficiency of the first metal wire.

In one embodiment, the ratio of the elastic energy storage efficiency of the first metal wire to the elastic energy storage efficiency of the second metal wire is in a range of 1:3 to 1:5. The ratio of the elastic energy storage efficiency of the third metal wire to the elastic energy storage efficiency of the second metal wire is in a range of 1:1.5 to 1:3.

In one embodiment, the ratio of a total length of the first cable body and the third cable body to the length of the second cable body is in a range of 1:2 to 1:20. The length ratio of the third cable body to the first cable body is in a range of 1:1 to 1:5.5.

In one embodiment, the second cable body is further provided with a push assistance portion.

In one embodiment, the push assistance portion includes a braid layer. The braid layer has a concave-convex outer surface.

In one embodiment, the push assistance portion includes a plurality of abutting members. The abutting members are spaced apart along a length direction of the second metal wire.

In one embodiment, the abutting member includes a sleeve surrounding the second metal wire. Alternatively, the abutting member includes a groove structure.

In one embodiment, the braid layer is further externally provided with a film layer. The film layer wraps the braid layer, and the film layer is formed with a concave-convex outer surface by the braid layer.

In one embodiment, the first cable body sequentially includes a first sub-section and a second sub-section connected to the first sub-section from the distal end to the proximal end. A distal end of the second sub-section is connected to the first sub-section, and a proximal end of the second sub-section is connected to the second cable body. The radial dimension of the distal end of the second sub-section is less than the radial dimension of the proximal end of the second sub-section.

In one embodiment, the braid layer includes a spring tube surrounding the second cable body. The length of the film layer is greater than that of the spring tube. A distal end of the film layer extends beyond a distal end of the spring tube.

In one embodiment, the distal end of the film layer tightly wraps an outer surface of the second cable body.

A push system includes a handle, a connector, and the push cable according to any one of the foregoing. The distal end of the first cable body is connected to the connector, and a proximal end of the second cable body is connected to the handle.

A heat treatment method includes:

   providing a to-be-treated metal member, where the to-be-treated metal member is made of a shape memory alloy material, and the elastic energy storage efficiency of the to-be-treated metal member is $\eta_1$; and

   placing the to-be-treated metal member in a heat treatment environment of 360°C to 430°C for 5 to 60 minutes, and then cooling the to-be-treated metal member to be less than or equal to 200°C within 50 to 120 minutes to obtain a treated metal member.

The elastic energy storage efficiency of the treated metal member is $\eta_2$, $\eta_1$ and $\eta_2$ are both greater than 0, and $\eta_2$ is less than $\eta_1$.

A push cable preparation method includes:

   providing a to-be-treated cable body; and

   performing heat treatment on one or more sections of the to-be-treated cable body based on the heat treatment method as described above to obtain a push cable, where the push cable includes a first cable body and a second cable body connected to one end of the first cable body, the first cable body includes one or more first metal wires, the second cable body includes one or more second metal wires, the elastic energy storage efficiency of the first metal wire is less than the elastic energy storage efficiency of the second metal wire, and in the environment of a human body temperature, when the first cable body is experiencing bending deformation from an initial form, the first cable body is not restored to the initial form without the assistance of an external force.

A push cable preparation method includes:

   preparing a first cable body and a second cable body, where the first cable body includes one or more first metal wires, the second cable body includes one or more second metal wires, the elastic energy storage efficiency of the first metal wire is less than the elastic energy storage efficiency of the second metal wire, and in the environment of a human body temperature, when the first cable body is experiencing bending deformation from an initial form, the first cable body is not restored to the initial form without the assistance of an external force; and

   fixedly connecting a proximal end of the first cable body to a distal end of the second cable body to obtain a push cable.

The push cable includes a first cable body and a second cable body connected to the first cable body. In the environment of a human body temperature, the first cable body is experiencing bending deformation from an initial form to release an occluder, and is not restored to the initial form without the assistance of an external force when the distal end of the push cable is disconnected from the occluder. Therefore, the probability of the first cable body flicking other parts of the heart is reduced, thereby reducing the probability of damage to heart tissue.

**Brief Description of the Drawings**

   FIG. 1 is a schematic view depicting a structure of a push system according to one embodiment of the present disclosure;

   FIG. 2 is a schematic view depicting a bending deformation restoration process of a push cable sample;

   FIG. 3 is a schematic view depicting a state in which a push cable in FIG. 1 is connected to an atrial septal defect occluder;

   FIG. 4 is a schematic view depicting a stress-strain curve of a push cable with a first metal wire in FIG. 1;

   FIG. 5 is a schematic view depicting a stress-strain curve of a push cable with a second metal wire in FIG. 1;

   FIG. 6 is a schematic view depicting a structure of a push cable according to another embodiment of the present disclosure;

   FIG. 7 is a schematic view depicting a structure of a push cable according to still another embodiment of the present disclosure;

   FIG. 8 is a schematic view depicting a structure of a push cable according to still another embodiment of the present disclosure;

FIG. 9 is a partial enlarged view of the push cable in FIG. 8;

FIG. 10 is a partial enlarged view of a push cable according to another embodiment of the present disclosure;

FIG. 11 is a schematic view of a heat treatment method according to an embodiment of the present disclosure;

FIG. 12 is a schematic view of a push cable preparation method according to an embodiment of the present disclosure;

FIG. 13 is a schematic view of a push cable preparation method according to another embodiment of the present disclosure;

FIG. 14 is a schematic view depicting a structure of a push cable according to still another embodiment of the present disclosure;

FIG. 15 is a schematic view depicting structures of a first cable body, a second cable body, and a push assistance portion according to still another embodiment of the present disclosure; and

FIG. 16 is a schematic enlarged view of region D in FIG. 14.

## Detailed Description of the Disclosure

In order to make the above-mentioned objects, features and advantages of the present disclosure more readily understood, the detailed description of the embodiments of the present disclosure will be given below with reference to the accompanying drawings. Many specific details are set forth in the following description to facilitate a full understanding of the present disclosure. However, the present disclosure can be practiced in many other ways different from those described herein, and similar improvements can be made by those skilled in the art without departing from the connotation of the present disclosure, whereby the present disclosure is not limited by the specific embodiments disclosed below.

Unless otherwise defined, all technical and scientific terms used herein have the same meaning as would normally be understood by those skilled in the art of the present disclosure. Terms used herein in the description of the present disclosure are for the purpose of describing specific embodiments only and are not intended to limit the present disclosure.

For a clearer description of the structure of the present disclosure, "distal end" and "proximal end" are used as locative terms, which are idiomatic terms in the field of interventional medical instruments, where the "distal end" represents the end away from an operator during surgery, and the "proximal end" represents the end near the operator during surgery.

Embodiment 1

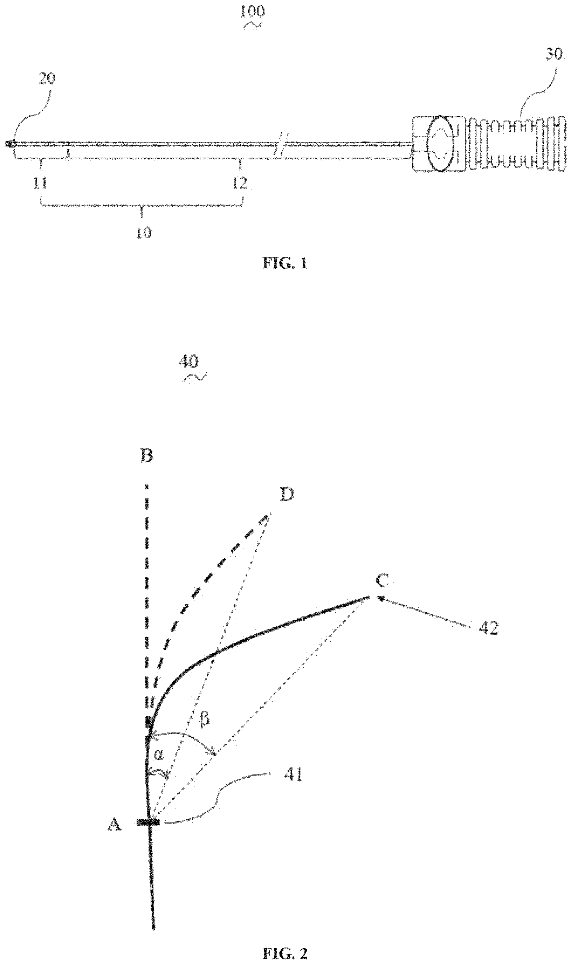

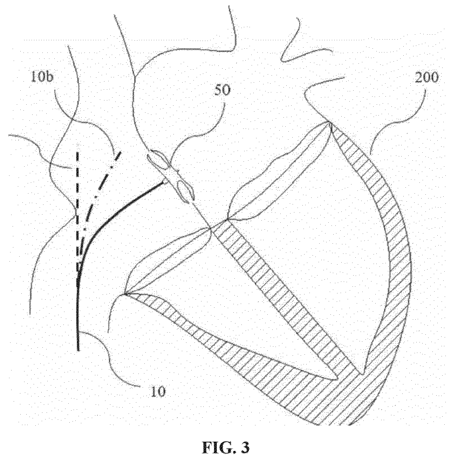

Referring to FIG. 1, this embodiment provides a push cable 10 applicable to a push system 100 for delivering an interventional medical instrument such as an occluder 50 for treating congenital heart diseases (referring to FIG. 3).

The push cable 10 includes a first cable body 11 and a second cable body 12. A distal end of the first cable body 11 is connected to a connector 20 for connecting the interventional medical instrument (such as the occluder 50, referring to FIG. 3). A proximal end of the first cable body 11 is connected to a distal end of the second cable body 12. A proximal end of the second cable body 12 is connected to a handle 30.

In this embodiment, the first cable body 11 and the second cable body 12 are integrally formed. Alternatively, the first cable body 11 is fixedly connected to the second cable body 12. For example, one end of the first cable body 11 is fixedly connected to one end of the second cable body 12 by welding. The first cable body 11 includes a single first metal wire, and the second cable body 12 includes a single second metal wire. The first metal wire and the second metal wire are each made of a shape memory alloy material (such as nickel-titanium alloy or nickel-titanium-copper triple alloy), have a solid structure, and have an equal-diameter circular cross section and a wire diameter (also referred to as a diameter or a radial dimension) of 0.5 mm to 1.0 mm. With the single metal wire structure of this embodiment, the push cable 10 of this embodiment has a smaller radial dimension and may be adapted to a delivery catheter of a smaller specification. Furthermore, with the solid structure of the metal wire, the push cable 10 has a sufficient push force to meet push requirements.

In other embodiments, the first cable body 11 may have a hollow tubular structure formed by spirally winding a plurality of first metal wires wound around a cylindrical mandrel and shaping, and alternatively, the first cable body 11 may have a solid structure formed by spirally winding a plurality of first metal wires. Similarly, the second cable body 12 may have a hollow tubular structure formed by spirally winding a plurality of second metal wires wound around the cylindrical mandrel and shaping, and alternatively, the second cable body 12 may have a solid structure formed by spirally winding a plurality of second metal wires. Circumscribed circles of the first cable body 11 and the second cable body 12 may have equal or unequal diameters. The diameters of the circumscribed circles of the first cable body 11 and the second cable body 12 are in a range of 1.0 to 4.5 mm.

It will be understood that the first cable body 11 and the second cable body 12 are not required to have the same structure in the present disclosure. Those skilled in the art may select appropriate structures for the first cable body 11 and the second cable body 12 according to specific application scenarios. Similarly, the present disclosure is not limited to the structures, materials, wire diameters, cross-sectional shapes, and other parameters of the first metal wire and the second metal wire. For example, the first metal wire and the second metal wire may also have a hollow structure. The cross sections of the first metal wire and the second metal wire may also be elliptical, triangular, quadrilateral, and the like. The wire diameters of the first metal wire and the second metal wire may be different, and the types of shape memory alloy materials used for the first metal wire and the second metal wire may also be different.

In this embodiment, when the first cable body 11 is experiencing bending deformation from an initial form in the environment of a human body temperature (36.0°C to 37.5°C), the first cable body 11 cannot be restored to the initial form without the assistance of an external force.

Referring to FIG. 2, a bending deformation restoration process of an elongated push cable sample 40 (hereinafter referred to as a sample 40) in FIG. 2 is, for example, simulated. An initial form of the sample is linear. The sample 40 is fixed to point A (fixed by, for example, a clamping device 41 shown in FIG. 2) in the environment of a human body temperature. An initial position of a free end 42 of the sample 40 is point B, and a distance from point A to point B is 80 mm (in practice, the distance from point A to point B may be any other suitable length). The sample 40 is applied with force such that the free end 42 of the sample 40 moves to point C. At this moment, the sample 40 is in a bent form, and a bending angle of the sample relative to the initial form is $\alpha$ (in this embodiment, $\alpha$ is an included angle between a connecting line AB and a connecting line AC). The application of force to the free end 42 of the sample 40 is stopped. The sample 40 undergoes the deformation restoration process and finally stops. At this moment, the sample 40 is in a restoration form. The free end 42 of the sample 40 is located at point D.

The restoration form of an existing push cable made of a shape memory alloy material is consistent with the initial form. When the push cable is in the restoration form, point D coincides with point B, and a deflection included angle $\beta$ between the restoration form and the initial form (in this embodiment, $\beta$ is an included angle between the connecting line AB and a connecting line AD) is equal to 0. When the first cable body 11 of this embodiment is applied to the above-mentioned simulation test, the deflection angle $\beta$ between the restoration form and the initial form is greater than 0, which means that the first cable body 11 has an irrecoverable deformation. Without the assistance of an external force, the first cable body 11 cannot be restored to the initial form, and the free end 42 of the first cable body 11 cannot be restored to point B. That is, the first cable body 11 has undergone plastic bending deformation.

Referring to FIG. 3, in this embodiment, when the first cable body 11 is connected to the occluder 50 and the occluder 50 is released, the first cable body 11 is in a first bending position (referring to a position of the push cable 10 represented by a solid line in FIG. 3). When the first cable body 11 is disconnected from the occluder 50, the first cable body 11 cannot restore to an initial position 10a, but can only be restored to a second bending position 10b which deviates from the initial position by a certain angle. Therefore, the probability of the first cable body 11 flicking other parts of the heart 200 (referring to FIG. 3) is reduced, thereby reducing the probability of damage to heart tissue.

Further, referring again to FIG. 2, for the same sample 40, there is a maximum plastic bending angle M (namely, a maximum of the included angle $\beta$). When the bending angle $\alpha$ of bending deformation is greater than the maximum plastic bending angle M, the included angle $\beta$ of the restoration form is equal to M. When the bending angle $\alpha$ of bending deformation is less than or equal to the maximum plastic bending angle M, the included angle $\beta$ of the restoration form is equal to $\alpha$. Therefore, when measuring the maximum plastic bending angle M of a sample 40, the angle may be obtained by multiple bending tests. The bending angle $\alpha$ of the bending test is gradually increased, and the included angle $\beta$ of the sample 40 corresponding to each bending angle $\alpha$ is recorded. When the measured included angle $\beta$ is less than the corresponding bending angle $\alpha$, the included angle $\beta$ is taken as the maximum plastic bending angle M of the sample 40. Alternatively, when the bending angle $\alpha$ is continuously increased and the corresponding included angle $\beta$ is no longer increased, the maximum of the included angle $\beta$ is taken as the maximum plastic bending angle M of the sample 40.

By measuring the maximum plastic bending angle M of an object, a maximum deformation of the object that cannot be restored to may be characterized. A larger maximum plastic bending angle M corresponds to a smaller bending restorability of the object, where the bending restorability refers to the capability of the object to be restored to its initial form without a force from bending deformation under the action of the force.

Regardless of the structure of the cable body of the push cable 10, the elastic energy storage efficiency of a shape memory alloy material (the elastic energy storage efficiency of the shape memory alloy material represents the ratio of the area of an unloading curve and the abscissa in a stress-strain curve of the shape memory alloy material to the area of a loading curve and the abscissa in the stress-strain curve) is associated with the maximum plastic bending angle of the cable body. A higher elastic energy storage efficiency of a shape memory alloy corresponds to a smaller maximum plastic bending angle (a stronger bending restorability) of a cable body made of the shape memory alloy. On the contrary, a lower elastic energy storage efficiency of a shape memory alloy corresponds to a larger maximum plastic bending angle (a weaker bending restorability) of a cable body made of the shape memory alloy. Therefore, the plastic bending angle of the cable body may be adjusted by controlling the elastic energy storage efficiency of the shape memory alloy material used for the cable body.

Taking the first metal wire and the second metal wire of this embodiment as examples, a method for measuring elastic energy storage efficiency is described in detail as follows:

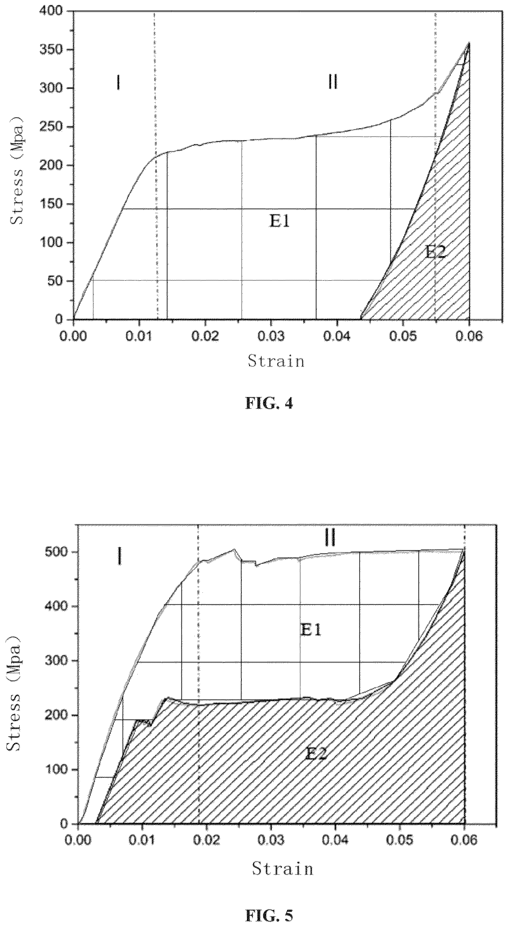

The first metal wire and the second metal wire are subjected to tension testing respectively, and a tension speed and a gauge length thereof may be performed according to ASTM F2516 Tension Testing of Nickel-Titanium Superelastic Materials. The tension speed is 4 mm/min, and the gauge length is 50 mm. A stress-strain curve of the first metal wire (referring to FIG. 4) and a stress-strain curve of the second metal wire (referring to FIG. 5) are obtained.

The elastic energy storage efficiency may be obtained by the following calculation formula:

$$\eta = E2/(E1+E2)$$

In the formula: $\eta$ represents the elastic energy storage efficiency.

E1 represents the energy density consumed per unit volume of materials during a stress-strain cycle.

E2 represents the energy density per unit volume of materials during unloading.

The value of E1 may be obtained by calculating the area of a part E1 (the part filled with lattices in FIG. 4 and FIG. 5) in a stress-strain curve graph, and the value of E2 may be obtained by calculating the area of a part E2 (the part filled with twills in FIG. 4 and FIG. 5) in the stress-strain curve graph. For example, the area may be calculated by some drawing software (such as original software).

In this embodiment, the elastic energy storage efficiency of the first metal wire is lower than the elastic energy storage efficiency of the second metal wire.

Further, the elastic energy storage efficiency of the first metal wire is not the case of smaller being better, as the extremely small elastic energy storage efficiency of the first metal wire is not conducive to the transmission of a push force of the first cable body 11 and affects the smoothness of push. Therefore, all the performances should be considered comprehensively to reasonably set the elastic energy storage efficiency of the first metal wire and the elastic energy storage efficiency of the second metal wire. The ratio of the elastic energy storage efficiency of the first metal wire to the elastic energy storage efficiency of the second metal wire may be in a range of 1:1.5 to 1:5. In other embodiments, the ratio of the elastic energy storage efficiency of the first metal wire to the elastic energy storage efficiency of the second metal wire may be in a range of 1:1.5 to 1:3 or 1:3 to 1:5. Therefore, the maximum plastic bending angle of the first cable body 11 is in a range of about 30° to 90°, the maximum plastic bending angle of the second cable body 12 is less than or equal to 10°, and both the first cable body 11 and the second cable body 12 can better transmit the push force, thereby improving the smoothness of the push. In other embodiments, the maximum plastic bending angle of the first cable body 11 may be in a range of 30° to 120°.

Furthermore, the lengths of the first cable body 11 and the second cable body 12 also determine the comprehensive performance of the push cable 10. The length ratio of the first cable body 11 to the second cable body 12 may be in a range of 1:2 to 1:20. The length ratio of the first cable body 11 to the second cable body 12 may also be in a range of 1:2 to 1:10 or 1:10 to 1:20. In this embodiment, the length of the first cable body 11 may be 80 mm to 130 mm, and the length of the second cable body 12 may be 270 mm to 1600 mm. In other embodiments, the length of the first cable body 11 may be 80 mm to 100 mm or 100 mm to 130 mm, and the length of the second cable body 12 may be 270 mm to 1000 mm or 1000 mm to 1600 mm.

In this embodiment, by reasonably setting the length relationship between the first cable body 11 and the second cable body 12, the probability of damage to heart tissue caused by disconnecting the push cable 10 from the occluder 50 can be further reduced, and it can be ensured that the push cable 10 is adapted to various bent lumens and better transmits the push force, so as to improve the smoothness of push.

Embodiment 2

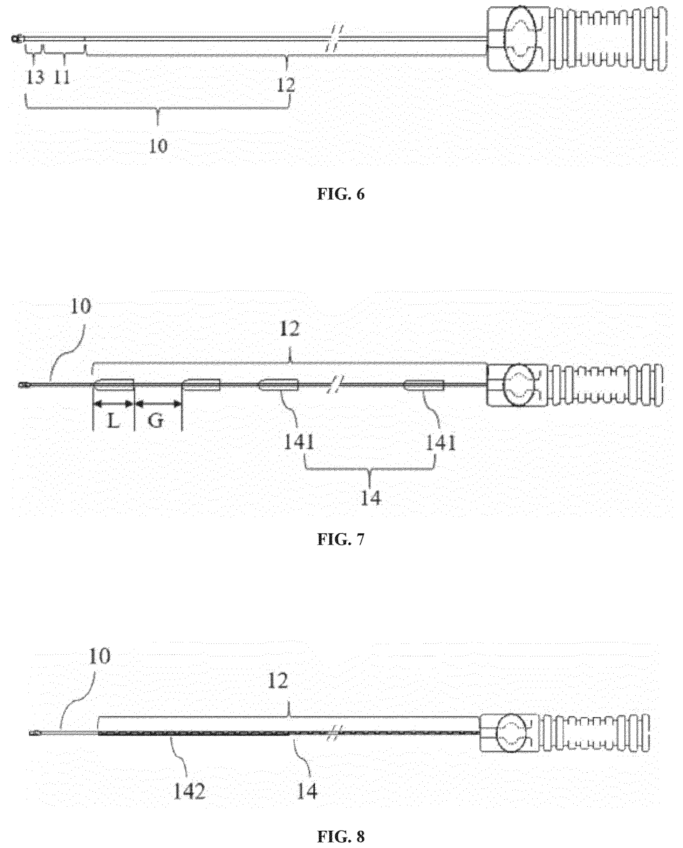

Referring to FIG. 6, this embodiment differs from Embodiment 1 in that the push cable 10 of this embodiment further includes a third cable body 13. The proximal end of the second cable body 12 is connected to the handle 30. The distal end of the second cable body 12 is connected to the proximal end of the first cable body 11. The distal end of the first cable body 11 is connected to a proximal end of the third cable body 13. A distal end of the third cable body 13 may be connected to the connector 20 for connecting the interventional medical instrument.

In this embodiment, the first cable body 11 and the third cable body 13 are integrally formed. Alternatively, the first cable body 11 is fixedly connected to the third cable body 13. For example, one end of the first cable body 11 is fixedly connected to one end of the third cable body 13 by welding. The first cable body 11 includes a single first metal wire, the second cable body 12 includes a single second metal wire, and the third cable body 13 includes a single third metal wire. The first metal wire, the second metal wire, and the third metal wire are each made of a shape memory alloy material, have a solid structure, and have an equal-diameter circular cross section and a wire diameter of 0.5 to 1.0 mm. With the single metal wire structure of this embodiment, the push cable 10 of this embodiment has a smaller radial dimension and may be adapted to a delivery catheter of a smaller specification. Furthermore, with the solid structure of the metal wire, the push cable 10 has a sufficient push force to meet push requirements.

In other embodiments, the first cable body 11 and the second cable body 12 may have other structures as exemplified in Embodiment 1. Similarly, the third cable body 13 may have a hollow tubular structure formed by spirally winding a plurality of third metal wires around a cylindrical mandrel and shaping. Alternatively, the third cable body 13 may have a solid structure formed by spirally winding a plurality of third metal wires. Circumscribed circles of the first cable body 11, the second cable body 12, and the third cable body 13 may have equal or unequal diameters. The diameters of the circumscribed circles of the first cable body 11, the second cable body 12, and the third cable body 13 are in a range of 1.0 to 4.5 mm.

It will be understood that the first cable body 11, the second cable body 12, and the third cable body 13 are not required to have the same structure in the present disclosure. Those skilled in the art may select appropriate structures for the first cable body 11, the second cable body 12, and the third cable body 13 according to specific application scenarios. Similarly, the present disclosure is not limited to the structures, materials, wire diameters, cross-sectional shapes, and other parameters of the first metal wire, the second metal wire, and the third metal wire.

In this embodiment, the elastic energy storage efficiency of the third metal wire is less than the elastic energy storage efficiency of the second metal wire and greater than the elastic energy storage efficiency of the first metal wire. The ratio of the elastic energy storage efficiency of the first metal wire to the elastic energy storage efficiency of the second metal wire may be in a range of 1:3 to 1:5. The ratio of the elastic energy storage efficiency of the third metal wire to the elastic energy storage efficiency of the second metal wire may be in a range of 1:1.5 to 1:3. In other embodiments, the ratio of the elastic energy storage efficiency of the first metal wire to the elastic energy storage efficiency of the second metal wire may also be in a range of 1:1.5 to 1:5. The ratio of the elastic energy storage efficiency of the third metal wire to the elastic energy storage efficiency of the second metal wire may also be in a range of 1:1.5 to 1:5. However, the value of the elastic energy storage efficiency of the third metal wire should be greater than the value of the elastic energy storage efficiency of the first metal wire.

In this embodiment, the ratio of the total length of the first cable body 11 and the third cable body 13 to the length of the second cable body 12 is in a range of 1:2 to 1:10 or 1:10 to 1:20. In this embodiment, the total length of the first cable body 11 and the third cable body 13 may be 80 mm to 100 mm or 100 mm to 130 mm, and the length of the second cable body 12 may be 270 mm to 1000 mm or 1000 mm to 1600 mm. The length ratio of the third cable body 13 to the first cable body 11 may also be in a range of 1:1 to 1:2.3 or 1:2.3 to 1:5.5. In this embodiment, the length of the first cable body 11 may be 40 mm to 70 mm or 70 mm to 110 mm, and the length of the third cable body 13 may be 20 mm to 30 mm or 30 mm to 40 mm.

The first cable body 11 is subjected to greater bending deformation than the third cable body 13 after the occluder 50 is released and before the push cable 10 of this embodiment is not disconnected from the occluder 50. Since the elastic energy storage efficiency of the first metal wire is greater than the elastic energy storage efficiency of the third metal wire, when the third cable body 13 is disconnected from the occluder 50, the first cable body 11 does not drive the third cable body 13 back to the initial position, so as to reduce the probability of the third cable body 13 flicking other parts of the heart 200 (referring to FIG. 3), thereby reducing the probability of damage to heart tissue. Furthermore, the elastic energy storage efficiency of the third metal wire is appropriately increased, whereby the farthest third cable body 13 can better transmit the push force to the interventional medical instrument. Therefore, by reasonably setting the elastic energy storage efficiency and the length relationship of the first metal wire, the second metal wire, and the third metal wire, the third cable body 13 may be used as a farthest part of the push cable 10, thereby better transmitting the push force to the occluder 50, and improving the smoothness of push.

Embodiment 3

When the second metal wire in Embodiments 1 and 2 has a single metal wire structure, an operator may find it difficult to hold the second metal wire during the push process due to the small wire diameter and the smooth outer surface of the second metal wire. The second metal wire can easily slip during the push process, and the push process can therefore be difficult. Based on this, referring to FIG. 7, the second cable body 12 of this embodiment is further provided with a push assistance portion 14 on the basis of Embodiments 1 and 2. The push assistance portion 14 includes a plurality of abutting members 141. The abutting members 141 are spaced apart along a length direction of the second cable body 12. During operation, the operator may select the abutting members 141 as abutting points one by one from the distal end to the proximal end of the second cable body 12. Then, the second cable body 12 is held, and a push force is applied to the push cable 10 against the selected abutting member 141. After one push is completed, the next abutting member 141 is selected as the abutting point for push. In this process, the abutting members 141 provide points of application of force for the operator, thus improving the push performance. Furthermore, the spaced apart abutting members 141 may also prompt the operator to push at a better distance each time, and the operator only needs to sequentially select the abutting members 141 to push, thus improving the convenience of the operation.

In this embodiment, the abutting member 141 is a sleeve that surrounds the second cable body 12. The sleeve tightly wraps the second cable body 12, such that the sleeve cannot be easily moved relative to the second cable body 12 when being applied with a push force by the operator.

Specifically, the sleeve may be formed by heat-shrinking a fluorinated ethylene propylene (FEP) heat-shrinkable tube. The specific preparation method includes: surrounding the second cable body 12 by the entire FEP heat-shrinkable tube; hot-melting the FEP heat-shrinkable tube to the second cable body 12 by hot-melt equipment; and after the hot melt is finished, performing sectional processing by using cutting tools (such as scalpel blades) according to dimensions to obtain multi-section FEP sleeves.

The sleeve has a proximal end face and a distal end face opposite to each other. During the push, the operator may hold the bare second cable body 12 between two adjacent sections of the sleeve with fingers, and push by pressing the fingers against the proximal end face of the farther sleeve.

In this embodiment, radial dimensions (including inner diameter dimensions and outer diameter dimensions) of the sleeve from the proximal end to the distal end are substantially the same. The ratio r of the outer diameter to the inner diameter affects the performance of the sleeve. A larger value of r means that there is a relatively larger area of the distal end face against which the operator abuts, so that it is more convenient for the operator to push with force. Furthermore, a larger value of r means that there is a higher integral rigidity of the sleeve sections, and a stronger support force may be provided for the push of the occluder 50. However, if the value of r is extremely large, the sleeve sections will not be easily bent, and the push cable 10 may be difficult to pass through a path with a large degree of bending. In this regard, the value of r should definitely not be extremely small. If the value of r is extremely small, the area of the distal end face against which the operator abuts will be extremely small. Therefore, the ratio of the outer diameter to the inner diameter is controlled in a range of 4:3 to 7:3, which is not only convenient for the operator to push, but also ensures that the second cable body 12 has a better bending performance and more suitable stiffness. Specifically, in this embodiment, the inner diameter of the sleeve may be set to 0.8-1.0 mm, and the outer diameter may be set to 1.2-2.2 mm.

In this embodiment, the sleeves have equal axial lengths, and the gaps between adjacent sleeves are equal too. The ratio S of an axial length L of a single sleeve to a length G of a single gap affects the performance of the push cable 10. On the one hand, a larger value of S corresponds to a relatively larger axial length L of the sleeve, which is beneficial to improve the rigidity of the second cable body 12. Furthermore, a larger value of S is beneficial to better transmit the torsional force at the proximal end of the push cable 10 to the distal end, thus avoiding relative torsion between the proximal end and the distal end of the push cable 10 due to the lag of torsional force transmission. When the push cable 10 is disconnected from the occluder 50, the distal end of the bent push cable 10 will rotate irregularly and scratch the inner wall of the heart 200. On the other hand, a smaller value of S corresponds to a relatively smaller axial length of the sleeve, which is beneficial to improve the bending performance of the second cable body 12. Furthermore, a smaller value of S also makes the gaps between the sleeves larger, which is convenient for the operator to push with the fingers therein. To sum up, by controlling the value of S in a range of 1-2, the second cable body 12 may have a stronger rigidity and a better bending performance, as well as a higher rotation force transmission efficiency. Furthermore, when the value of S is within this range, the single push distance for the operator does not exceed the sum of L and G. That is, the push distance is more suitable, whereby the second cable body 12 is prevented from bending due to a long push distance, and the low push efficiency due to a short push distance can be avoided.

Further, in this embodiment, the radius of the circumscribed circle of the distal end face of the sleeve may be greater than the radius of the circumscribed circle of the proximal end face (for example, the outer diameter of the sleeve is gradually increased from the proximal end to the distal end). By increasing the dimension of the distal end face, the area against which the operator abuts can be increased, which is convenient for the operator to push with force. A smaller proximal end face can reduce the push resistance caused by blood and other fluids to the push cable 10, whereby the push process is smoother.

Further, the outer surface of the sleeve may be smoothened by selecting a suitable material (for example, a polymer material such as PTFE and FEP with a small friction coefficient) to reduce friction between the push cable 10 and an inner wall of a delivery catheter and reduce the push resistance, thereby reducing the push difficulty of the instrument and effectively reducing the risk of tearing of an inner membrane of the catheter.

Further, the roughness of the second cable body 12 may be increased, whereby the roughness of the second cable body 12 is greater than that of the first cable body 11. The increase in the roughness of the second cable body 12 is not only beneficial to increase the friction between the second cable body and the sleeve, whereby the sleeve more tightly wraps the second cable body 12. In addition, it is easier for the operator to hold the second cable body 12, thus preventing slipping.

In other embodiments, the push assistance portion 14 may also be a plurality of spaced apart groove structures (not shown). The groove structures are spaced apart along a length direction of the second cable body 12. When pushing, the operator may hold the groove structures with the fingers and push with force.

Embodiment 4

Referring to FIG. 8, this embodiment differs from Embodiment 3 in that the push assistance portion 14 of this embodiment includes a braid layer 142. The second cable body 12 has a concave-convex outer surface through the braid layer 142.

The braid layer 142 may be formed by braiding metal wires made of nickel-titanium alloy or stainless steel and includes a plurality of convex units 1421 and a plurality of concave regions 1422 spaced apart.

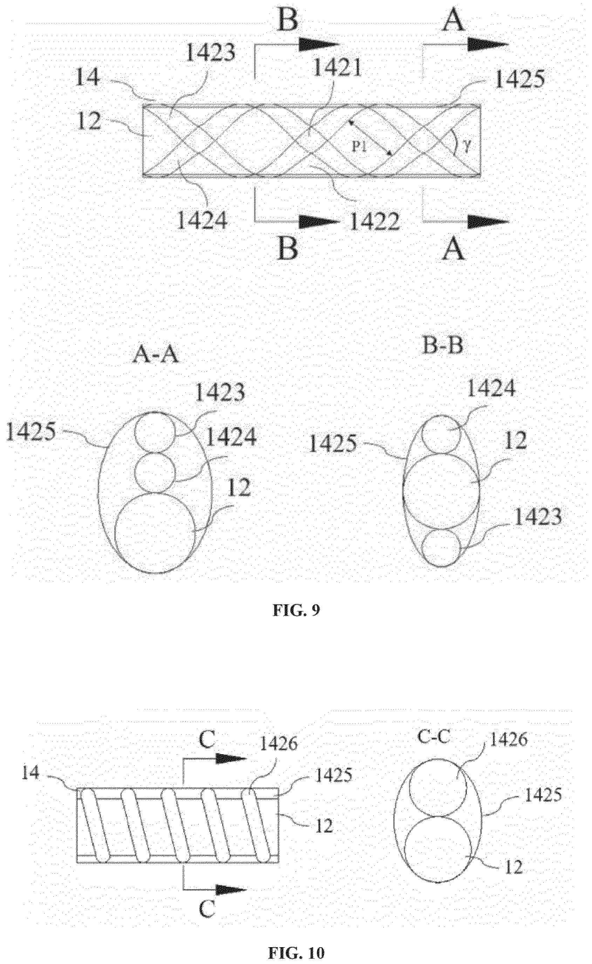

Referring to FIG. 9, the braid layer 142 includes a first braid wire 1423 and a second braid wire 1424. The first braid wire 1423 is spirally wound on the second cable body 12 in a first winding direction, and the second braid wire 1424 is spirally wound on the second cable body 12 in a second winding direction. The convex units 1421 are formed at the intersection of the first braid wire 1423 and the second braid wire 1424, and a portion of the outer surface not covered by the first braid wire 1423 and the second braid wire 1424 forms the concave regions 1422. Each concave region 1422 is enclosed by the first braid wire 1423 and the second braid wire 1424. On the one hand, the arrangement of the braid layer 142 provides the operator with a better holding feeling, and the operator can push smoothly by applying a small holding force, thus preventing slipping during the push process. On the other hand, it is beneficial to better transmit the torsional force at the proximal end of the push cable 10 to the distal end.

In FIG. 9, the first braid wire 1423 and the second braid wire 1424 are both circular in cross section and equal in diameter of 0.25 mm to 0.35 mm. Winding pitches P1 (pitch) of the first braid wire 1423 and the second braid wire 1424 are in a range of 4 mm to 8 mm, and a braid angle $\gamma$ (cross angle in a radial direction) between the first braid wire 1423 and the second braid wire 1424 is in a range of 60° to 90°. By reasonably setting the wire diameters of the first braid wire 1423 and the second braid wire 1424, the convex units 1421 are convex by 0.5 mm to 0.7 mm relative to the second cable body 12. That is, the concave regions 1422 have a radial concave dimension of 0.5 mm to 0.7 mm. Furthermore, by reasonably setting parameters such as the winding pitches and the braid angle of the first braid wire 1423 and the second braid wire 1424, the concave regions 1422 are ensured to have a sufficiently large area. It is beneficial to further improve the push efficiency of the operator, and the second cable body 12 has excellent rigidity and bending performance, whereby the occluder 50 can be smoothly pushed to the defect site.

Further, with continued reference to FIG. 9, in this embodiment, the braid layer 142 is further externally provided with a film layer 1425. The film layer 1425 wraps the braid layer 142, and the film layer 1425 is formed with a concave-convex outer surface by the braid layer 142. The thickness of the film layer 1425 should be appropriate. The film layer 1425 that is extremely thin is prone to damage during bending of the second cable body 12, and is not conducive to transmission of the torsional force. If the film layer 1425 is extremely thick, the concave-convex effect of the outer surface is less obvious, and the radial dimensions of the second cable body 12 may be extremely large. Therefore, the wall thickness of the film layer 1425 in this embodiment is 0.1 mm to 0.2 mm, which is beneficial to improve the comprehensive push performance of the push cable 10.

The film layer 1425 may be made of a polymer material, for example, one of PTFE and FEP, which has a low friction coefficient of 0.1 to 0.2, thereby reducing the friction between the push cable and an inner wall of a delivery catheter, reducing the push difficulty of the instrument and effectively reducing the risk of tearing of an inner membrane of the catheter.

Referring to FIG. 10, in other embodiments, the braid layer 142 may be formed by winding a single third braid wire 1426 on the second cable body 12 with a winding pitch P2 of 4 mm to 8 mm, and the wire diameter of the third braid wire 1426 is 0.55 mm to 0.65 mm. The braid method also provides a good push performance and holding feeling.

In other embodiments, the cross-sectional shapes of the first braid wire 1423, the second braid wire 1424, and the third braid wire 1426 may also be elliptical, triangular, quadrilateral, or other irregular shapes, which is beneficial to improve the winding firmness, prevent mutual sliding with the second cable body 12 and increase the concave-convex feeling of the outer surface of the second cable body 12 compared with the circular shape.

Embodiment 5

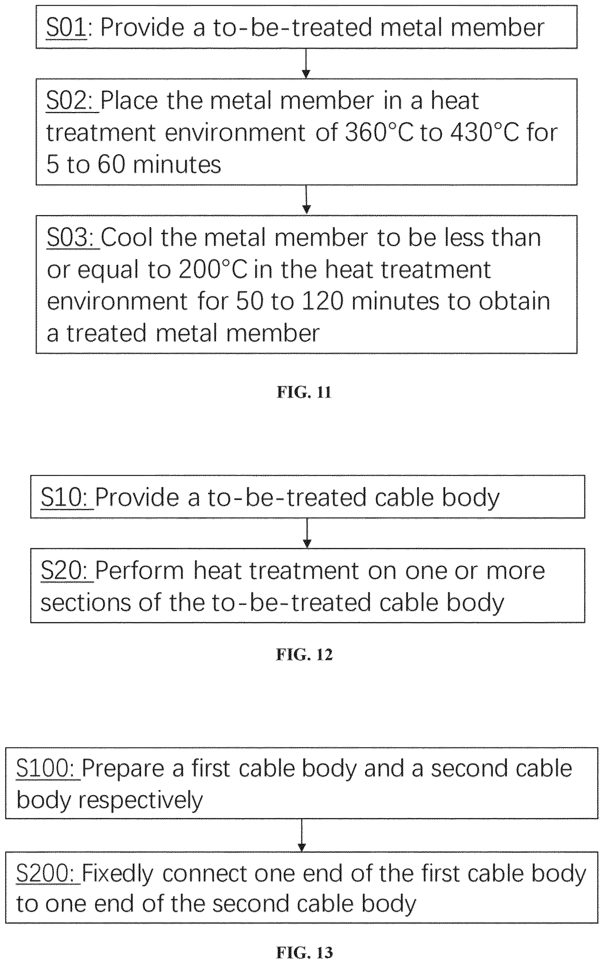

Referring to FIG. 11, this embodiment provides a heat treatment method. The method includes:
Step S01: Provide a to-be-treated metal member, the to-be-treated metal member is made of a shape memory alloy material, and the elastic energy storage efficiency of the to-be-treated metal member is $\eta_1$.

Specifically, the metal member includes one or more metal wires, and the metal member may also be one or more sections of an entire metal cable (the metal cable includes one or more metal wires). In other embodiments, the metal member may also be a metal rod, a metal block, or the like. The dimensions, shape and structure of the metal member are not limited by the present disclosure.

Step S02: Place the metal member in a heat treatment environment of 360°C to 430°C for 5 to 60 minutes.

Specifically, the metal member is placed in a heat treatment environment (such as a quartz tube), and the vacuum degree of the heat treatment environment is reduced to be less than or equal to 5 Pa. Then the heat treatment environment is heated. When the temperature is heated to 360°C-400°C or 400°C-420°C or 420°C-430°C, the on-off of induction heating equipment is controlled by temperature control equipment, and the heat treatment environment is kept at this temperature for 5 to 35 minutes or 35 to 60 minutes.

Step S03: Cool the metal member to be less than or equal to 200°C in the heat treatment environment for 50 to 120 minutes to obtain a treated metal member.

The vacuum degree of the heat treatment environment is kept to be less than or equal to 5 Pa, and the heat treatment environment is stopped from being heated, and is slowly cooled to be less than or equal to 200°C, the cooling time is about 50 to 80 minutes or 80 to 100 minutes or 100 to 120 minutes. Finally, the heat treatment environment is stopped from being vacuumized, and the heat treatment environment is slowly ventilated with air. When the air pressure reaches $1 \times 10^5$ Pa, the treated metal member is taken out.

The elastic energy storage efficiency of the treated metal member is $\eta_2$, $\eta_1$ and $\eta_2$ are both greater than 0, and $\eta_2$ is less than $\eta_1$.

The elastic energy storage efficiency of the first metal wire, the second metal wire, and the third metal wire in Embodiments 1-3 may be set and adjusted by the heat treatment method.

Embodiment 6

Referring to FIG. 12, this embodiment provides a push cable preparation method. The method includes: Step S10: Provide a to-be-treated cable body.

The to-be-treated cable body may include one or more metal wires made of a shape memory alloy material. The specific cable body structure may refer to the structural arrangement of the first cable body, the second cable body, and the third cable body described in Embodiments 1 and 2.

Step S20: Perform heat treatment on one or more sections of the to-be-treated cable body according to the heat treatment method in Embodiment 5 to obtain the push cable in Embodiments 1 and 2.

For example, the to-be-treated cable body is marked to sequentially mark a first cable body and a second cable body on the to-be-treated cable body, or the first cable body, the second cable body, and the third cable body are sequentially marked on the to-be-treated cable body, or more cable bodies may be sequentially marked on the to-be-treated cable body. It should be noted that in this step, the to-be-treated cable body only needs to be marked without being cut.

After a plurality of cable bodies are marked, one or more of the cable bodies are subjected to heat treatment as required. For example, after the to-be-treated cable body is marked as a first cable body and a second cable body, only the first cable body may be subjected to heat treatment and the second cable body may not be subjected to heat treatment to obtain the push cable in Embodiment 1. The first cable body and the second cable body may also be subjected to heat treatment respectively to obtain the push cable in Embodiment 1. After the to-be-treated cable body is marked as a first cable body, a second cable body, and a third cable body, the first cable body and the second cable body may be subjected to heat treatment respectively, or the first cable body and the second cable body may be subjected to heat treatment simultaneously. Then, one of the first cable body and the second cable body may be further subjected to heat treatment to further reduce the elastic energy storage efficiency of a metal wire in this cable body, while the third cable body is not subjected to heat treatment to obtain the push cable in Embodiment 2. The first cable body, the second cable body, and the third cable body may be subjected to heat treatment respectively to obtain the push cable in Embodiment 2. In summary, regardless of the number of cable bodies into which the to-be-treated cable body is marked, one or more cable bodies may be subjected to single or multiple heat treatments as required. One or more cable bodies may be taken as the metal member in Embodiment 5, and the heat treatment method may be performed according to the heat treatment method described in Embodiment 5.

Embodiment 7

Referring to FIG. 13, this embodiment provides a push cable preparation method. The method includes: Step S100: Prepare a first cable body and a second cable body respectively.

The first cable body includes one or more first metal wires, and the second cable body includes one or more second metal wires. The elastic energy storage efficiency of the first metal wire is less than the elastic energy storage efficiency of the second metal wire. In the environment of a human body temperature, when the first cable body is experiencing bending deformation from an initial form, the first cable body cannot restore to the initial form without the assistance of an external force. The specific shapes, structures, performances, and dimensions of the first cable body and the second cable body may refer to the first cable body and the second cable body in Embodiment 1.

Step S200: Fixedly connect one end of the first cable body to one end of the second cable body (by, for example, welding, adhering, and the like) to obtain the push cable in Embodiment 1.

This embodiment also provides another push cable preparation method. The method includes:
A first cable body, a second cable body, and a third cable body are prepared respectively. The shapes, structures, performances, and dimensions of the first cable body, the second cable body, and the third cable body may refer to the first cable body, the second cable body, and the third cable body in Embodiment 2. One end of the first cable body is fixedly connected to one end of the second cable body (by, for example, welding, adhering, and the like), and the other end of the first cable body is fixedly connected to one end of the third cable body (by, for example, welding, adhering, and the like) to obtain the push cable in Embodiment 2.

The preparation method of the cable body (for example, the first cable body, the second cable body, or the third cable body) may include: first, making a cable body preform by using a metal wire; if the elastic energy storage efficiency of the cable body preform needs to be adjusted, performing heat treatment on the cable body preform according to the heat treatment method in Embodiment 5 to obtain a finished cable body product; and if heat treatment is not needed, omitting the heat treatment steps, and directly using the cable body preform as the finished cable body product.

In other embodiments, the elastic energy storage efficiency of a metal material (such as a metal wire or a metal rod used for fabricating a metal wire) for fabricating a cable body (such as a first cable body, a second cable body, or a third cable body) may be adjusted in advance by adopting the heat treatment method in Embodiment 5 as required, and then a metal material with appropriate elastic energy storage efficiency may be selected for fabricating the cable body.

In other embodiments, the push cable may also be formed by connecting more than three cable bodies, and the appropriate elastic energy storage efficiency may be set for each cable body as required.

Embodiment 8

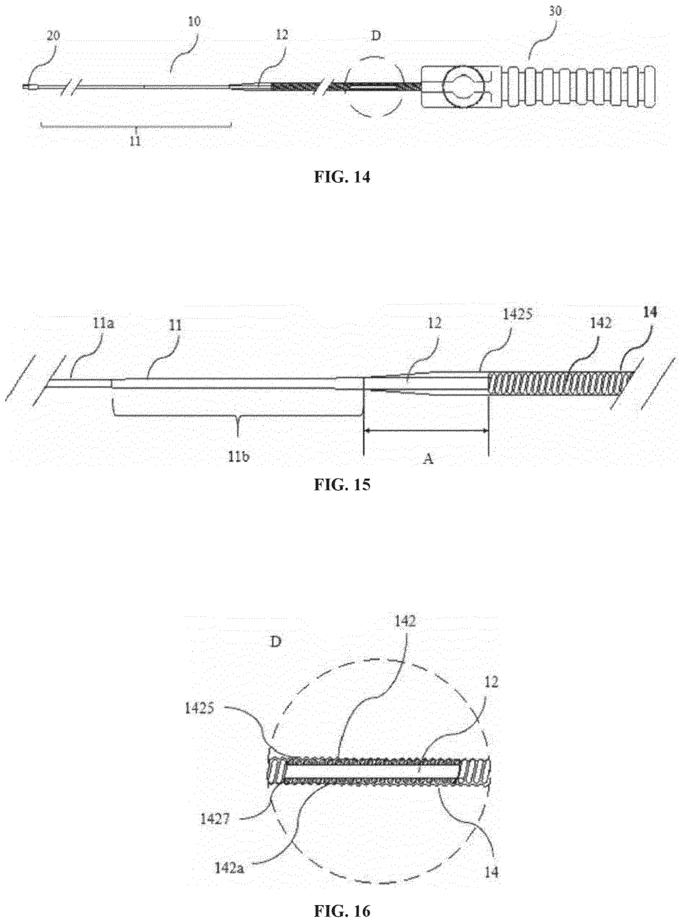

Referring to FIG. 14, this embodiment provides a push cable 10 applicable to a push system 100 for delivering an interventional medical instrument such as an occluder 50 for treating congenital heart diseases (referring to FIG. 3).

The push cable 10 includes a first cable body 11 and a second cable body 12. A distal end of the first cable body 11 is connected to a connector 20 for connecting the interventional medical instrument (such as the occluder 50, referring to FIG. 3). A proximal end of the first cable body 11 is connected to a distal end of the second cable body 12. A proximal end of the second cable body 12 is connected to a handle 30.

In this embodiment, the first cable body 11 and the second cable body 12 are integrally formed. Alternatively, the first cable body 11 is fixedly connected to the second cable body 12. For example, one end of the first cable body 11 is fixedly connected to one end of the second cable body 12 by welding. The first cable body 11 includes a single first metal wire, and the second cable body 12 includes a single second metal wire. The first metal wire and the second metal wire are each made of a shape memory alloy material (such as nickel-titanium alloy or nickel-titanium-copper triple alloy), have a solid structure, and have a substantially circular cross section and a wire diameter (also referred to as a diameter or a radial dimension) of 0.5 mm to 1.0 mm. With the single metal wire structure of this embodiment, the push cable 10 of this embodiment has a smaller radial dimension and may be adapted to a delivery catheter of a smaller specification. Furthermore, with the solid structure of the metal wire, the push cable 10 has a sufficient push force to meet push requirements.

In other embodiments, the first cable body 11 may have a hollow tubular structure formed by spirally winding a plurality of first metal wires wound around a cylindrical mandrel and shaping, and alternatively, the first cable body 11 may have a solid structure formed by spirally winding a plurality of first metal wires. Similarly, the second cable body 12 may have a hollow tubular structure formed by spirally winding a plurality of second metal wires wound around the cylindrical mandrel and shaping, and alternatively, the second cable body 12 may have a solid structure formed by spirally winding a plurality of second metal wires. Circumscribed circles of the first cable body 11 and the second cable body 12 may have equal or unequal diameters. The diameters of the circumscribed circles of the first cable body 11 and the second cable body 12 are in a range of 1.0 to 4.5 mm.

It will be understood that the first cable body 11 and the second cable body 12 are not required to have the same structure in the present disclosure. Those skilled in the art may select appropriate structures for the first cable body 11 and the second cable body 12 according to specific application scenarios. Similarly, the present disclosure is not limited to the structures, materials, wire diameters, cross-sectional shapes, and other parameters of the first metal wire and the second metal wire. For example, the first metal wire and the second metal wire may also have a hollow structure. The cross sections of the first metal wire and the second metal wire may also be elliptical, triangular, quadrilateral, and the like. The wire diameters of the first metal wire and the second metal wire may be different, and the types of shape memory alloy materials used for the first metal wire and the second metal wire may also be different.

In this embodiment, when the first cable body 11 is experiencing bending deformation from an initial form in the environment of a human body temperature (36.0°C to 37.5°C), the first cable body 11 cannot be restored to the initial form without the assistance of an external force. For example, in this embodiment, the elastic energy storage efficiency of the first metal wire is lower than the elastic energy storage efficiency of the second metal wire.

In this embodiment, in the environment of a human body temperature, the first cable body 11 is experiencing bending deformation from an initial form to release the occluder 50, and cannot be restored to the initial form without the assistance of an external force when the distal end of the push cable 10 is disconnected from the occluder 50. Therefore, the probability of the first cable body 11 flicking other parts of the heart is reduced, thereby reducing the probability of damage to heart tissue.

Furthermore, the lengths of the first cable body 11 and the second cable body 12 also determine the comprehensive performance of the push cable 10. The length ratio of the first cable body 11 to the second cable body 12 may be in a range of 1:2 to 1:20. The length ratio of the first cable body 11 to the second cable body 12 may also be in a range of 1:2 to 1:10 or 1:10 to 1:20. In this embodiment, the length of the first cable body 11 may be 80 mm to 130 mm, and the length of the second cable body 12 may be 270 mm to 1600 mm. In other embodiments, the length of the first cable body 11 may be 80 mm to 100 mm or 100 mm to 130 mm, and the length of the second cable body 12 may be 270 mm to 1000 mm or 1000 mm to 1600 mm.

In this embodiment, by reasonably setting the length relationship between the first cable body 11 and the second cable body 12, the probability of damage to heart tissue caused by disconnecting the push cable 10 from the occluder 50 can be further reduced, and it can be ensured that the push cable 10 is adapted to various bent lumens and better transmits the push force, so as to improve the smoothness of push.

The first cable body 11 of this embodiment sequentially includes a first sub-section 11a and a second sub-section 11b connected to the first sub-section 11a from the distal end to the proximal end. A distal end of the first sub-section 11a is connected to the connector 20. A proximal end of the first sub-section 11a is connected to a distal end of the second sub-section 11b. A proximal end of the second sub-section 11b is connected to the second cable body 12. The first sub-section 11a and the second sub-section 11b are integrally formed. In other embodiments, the first sub-section 11a and the second sub-section 11b may be respectively fabricated and spliced together.

The first sub-section 11a has a relatively uniform radial dimension, while the second sub-section 11b has a tapered structure. The proximal end of the second sub-section 11b has a radial dimension substantially equal to that of the second cable body 12. The distal end of the second sub-section 11b has a radial dimension substantially equal to that of the first sub-section 11a. The ratio of the radial dimension of the proximal end of the second sub-section 11b to the radial dimension of the distal end of the second sub-section 11b is in a range of 1.2 to 2.5. This arrangement not only enables the second cable body 12 to better transmit the push force, but also further reduces the probability of damage to heart tissue after the push cable 10 is disconnected from the occluder 50. The arrangement of the second sub-section 11b enables a smooth transition between the second cable body 12 and the first sub-section 11a, thus avoiding scratching biological tissue during the delivery process due to the generation of steps. Furthermore, the length ratio of the first sub-section 11a to the second sub-section 11b may be in a range of 1 to 5. For example, the length ratio may be 1, 2, 3, 3.5, 4, 5, or the like.

Further, in order to make the second cable body 12 easier to push during the push process, a push assistance portion 14 may be arranged on the second cable body 12. The push assistance portion 14 includes a braid layer 142. The braid layer 142 has a concave-convex outer surface.

The braid layer 142 may be formed by braiding metal wires made of nickel-titanium alloy or stainless steel, and includes a spring tube 142a. The spring tube 142a may be formed by winding a single fourth braid wire 1427 with a pitch of 0.1 mm to 8 mm, and the wire diameter of the fourth braid wire 1427 is 0.1 mm to 0.8 mm. For example, the wire diameter of the fourth braid wire 1427 may be 0.1 mm, 0.2 mm, 0.3 mm, 0.35 mm, 0.4 mm, 0.5 mm, 0.6 mm, 0.7 mm, or the like. In other embodiments, the spring tube 142a may also be formed by winding a plurality of fourth braid wires 1427 in parallel. The braid layer 142 may provide the second cable body 12 with a good push performance and holding feeling.

In this embodiment, the cross-sectional shape of the fourth braid wire 1427 is substantially circular. In other embodiments, the cross-sectional shape of the fourth braid wire may also be elliptical, triangular, quadrilateral, or other irregular shapes, which is beneficial to improve the winding firmness, prevent mutual sliding with the second cable body 12 and increase the concave-convex feeling of the outer surface of the second cable body 12 compared with the circular shape.

In this embodiment, the braid layer 142 is further externally provided with a tubular film layer 1425. The film layer 1425 wraps the braid layer 142, and the film layer 1425 is formed with a concave-convex outer surface by the braid layer 142. The film layer 1425 may be made of a polymer material such as PEBAX. To fabricate the film layer 1425, a PEBAX tube first surrounds the outside of the braid layer 142, and then a heat-shrinkable tube is surrounded outside the PEBAX tube. After heating, the heat-shrinkable tube shrinks and the PEBAX tube is hot-melted and penetrates into a threaded gap of the spring tube 142a. After cooling, the heat-shrinkable tube is peeled off, and the film layer 1425 is formed on the surface of the braid layer 142. Since the film layer 1425 is fitted into the threaded gap of the spring tube 142a, the spring tube 142a and the second cable body 12 can be tightly connected to prevent the spring tube 142a from sliding relative to the second cable body 12. At the same time, the film layer 1425 forms a concave-convex outer surface in compliance with the shape of the spring tube 142a. The thickness of the film layer 1425 should be appropriate. If the film layer 1425 is extremely thin, it is prone to damage during bending of the second cable body 12, and is not conducive to transmission of the torsional force. If the film layer 1425 is extremely thick, the concave-convex effect of the outer surface is less obvious, and the radial dimensions of the second cable body 12 may be extremely large. Therefore, the wall thickness of the film layer 1425 in this embodiment is 0.02 mm to 0.1 mm, which is beneficial to improve the comprehensive push performance of the push cable 10.

Further, the length of the film layer 1425 is larger than the length of the spring tube 142a. A distance A exists between the distal end of the spring tube 142a and the distal end of the second cable body 12. The ratio of the distance A to the length of the second cable body 12 is in a range of 1:130 to 1:90. The distal end of the film layer 1425 extends beyond the distal end of the spring tube 142a to the distal end of the second cable body 12. The outer diameter of the film layer 1425 at the distal end of the spring tube 142a is greater than the outer diameter of the distal end of the film layer 1425, and the distal end of the film layer 1425 tightly wraps the outer surface of the second cable body 12. In this way, the film layer 1425 can wrap steps formed between the distal end of the spring tube 142a and the second cable body 12 to prevent the steps from scratching biological tissue during the delivery. Furthermore, the distal end of the film layer 1425 tightly wraps the outer surface of the second cable body 12, so as not only to prevent damage to biological tissue, but also to prevent blood from penetrating between the film layer 1425 and the second cable body 12.

The push cable, the heat treatment method, and the push cable preparation method are further described below by specific examples.

Example 1

A solid nickel-titanium wire is provided, with a wire diameter of 1.0 mm, a length of 400 mm, and an elastic energy storage efficiency of 70%. One end of the nickel-titanium wire (having a length of 130 mm) is placed in a heat treatment furnace at a temperature of 430°C, and the remaining nickel-titanium wire (having a length of 270 mm) is placed outside the heat treatment furnace. After 5 minutes, the nickel-titanium wire is cooled to 200°C within 50 minutes, and then naturally cooled to room temperature to obtain a push cable. The elastic energy storage efficiency of the end subjected to heat treatment is 46%, and the elastic energy storage efficiency of the end not subjected to heat treatment is still 70%.

After the atrial septum of the heart of a healthy pig is punctured by a puncture system, the push cable is used in a delivery system, and the end subjected to heat treatment is used as a distal end of the push cable. An atrial septal defect occluder is delivered to an atrial septal defect of the heart of the pig. The push and release process is smooth, and after release, the distal end of the push cable is not restored to an initial form without obvious damage to heart tissue.

Example 2

A hollow nickel-titanium wire is provided, with an outer diameter of 0.8 mm, a length of 1680 mm, and an elastic energy storage efficiency of 65%. One end of the nickel-titanium wire (having a length of 80 mm) is placed in a heat treatment furnace at a temperature of 360°C, and the remaining nickel-titanium wire (having a length of 1600 mm) is placed outside the heat treatment furnace. After 60 minutes, the nickel-titanium wire is cooled to 200°C within 120 minutes, and then naturally cooled to room temperature to obtain a push cable. The elastic energy storage efficiency of the end subjected to heat treatment is 21%, and the elastic energy storage efficiency of the end not subjected to heat treatment is still 65%.

After the ventricular septum of the heart of a healthy pig is punctured by a puncture system, the push cable is used in a delivery system, and the end subjected to heat treatment is used as a distal end of the push cable. A ventricular septal defect occluder is delivered to a ventricular septal defect of the heart of the pig. The push and release process is smooth, and after release, the distal end of the push cable is not restored to an initial form without obvious damage to heart tissue.

Example 3

A solid nickel-titanium wire is provided, with a wire diameter of 0.5 mm, a length of 1100 mm, and an elastic energy storage efficiency of 80%. One end of the nickel-titanium wire (having a length of 100 mm) is placed in a heat treatment furnace at a temperature of 400°C, and the remaining nickel-titanium wire (having a length of 1000 mm) is placed outside the heat treatment furnace. After 40 minutes, the nickel-titanium wire is cooled to 180°C within 100 minutes, and then naturally cooled to room temperature to obtain a push cable. The elastic energy storage efficiency of the end subjected to heat treatment is 16%, and the elastic energy storage efficiency of the end not subjected to heat treatment is still 80%.

After the ventricular septum of the heart of a healthy pig is punctured by a puncture system, the push cable is used in a delivery system, and the end subjected to heat treatment is used as a distal end of the push cable. A ventricular septal defect occluder is delivered to a ventricular septal defect of the heart of the pig. The push and release process is smooth, and after release, the distal end of the push cable is not restored to an initial form without obvious damage to heart tissue.

Example 4

A solid nickel-titanium wire is provided, with a wire diameter of 1.0 mm, a length of 400 mm, and an elastic energy storage efficiency of 70%. The nickel-titanium wire is marked so as to sequentially mark a first section of the nickel-titanium wire, a second section of the nickel-titanium wire, and a third section of the nickel-titanium wire on the nickel-titanium wire. Both ends of the second section of the nickel-titanium wire are connected to the first section of the nickel-titanium wire and the third section of the nickel-titanium wire respectively.

The first section of the nickel-titanium wire has a length of 20 mm, the second section of the nickel-titanium wire has a length of 110 mm, and the third section of the nickel-titanium wire has a length of 270 mm. The first section of the nickel-titanium wire and the second section of the nickel-titanium wire are subjected to heat treatment respectively, and the third section of the nickel-titanium wire is not subjected to heat treatment. The heat treatment process of the first section of the nickel-titanium wire includes: placing the first section of the nickel-titanium wire in a heat treatment furnace at a temperature of 430°C, keeping for 5 minutes, cooling to 200°C within 50 minutes, and then naturally cooling to room temperature. The heat treatment process of the second section of the nickel-titanium wire includes: placing the second section of the nickel-titanium wire in a heat treatment furnace at a temperature of 420°C, keeping for 35 minutes, cooling to 200°C within 80 minutes, and then naturally cooling to room temperature. After heat treatment of the first section of the nickel-titanium wire and the second section of the nickel-titanium wire, a push cable is obtained. The elastic energy storage efficiency of the first section of the nickel-titanium wire subjected to heat treatment is 46%, the elastic energy storage efficiency of the second section of the nickel-titanium wire subjected to heat treatment is 17.5%, and the elastic energy storage efficiency of the third section of the nickel-titanium wire not subjected to heat treatment is still 70%.

After the atrial septum of the heart of a healthy pig is punctured by a puncture system, the push cable is used in a delivery system, and the first section of the nickel-titanium wire is connected to an atrial septal defect occluder. The atrial septal defect occluder is delivered to an atrial septal defect of the heart of the pig. The push and release process is smooth, and after the atrial septal defect occluder is released, the distal end of the push cable is not restored to an initial form without obvious damage to heart tissue.

Example 5

A hollow nickel-titanium wire is provided, with a wire diameter of 0.8 mm, a length of 1680 mm, and an elastic energy storage efficiency of 65%. The nickel-titanium wire is marked so as to sequentially mark a first section of the nickel-titanium wire, a second section of the nickel-titanium wire, and a third section of the nickel-titanium wire on the nickel-titanium wire. Both ends of the second section of the nickel-titanium wire are connected to the first section of the nickel-titanium wire and the third section of the nickel-titanium wire, respectively. The first section of the nickel-titanium wire has a length of 40 mm, the second section of the nickel-titanium wire has a length of 40 mm, and the third section of the nickel-titanium wire has a length of 1600 mm. The first section of the nickel-titanium wire and the second section of the nickel-titanium wire are subjected to heat treatment respectively, and the third section of the nickel-titanium wire is not subjected to heat treatment. The heat treatment of the first section of the nickel-titanium wire includes: placing the first section of the nickel-titanium wire in a heat treatment furnace at a temperature of 420°C, keeping for 20 minutes, cooling to 200°C within 60 minutes, and then naturally cooling to room temperature. The heat treatment of the second section of the nickel-titanium wire includes: placing the second section of the nickel-titanium wire in a heat treatment furnace at a temperature of 360°C, keeping for 60 minutes, cooling to 200°C within 120 minutes, and then naturally cooling to room temperature. After heat treatment of the first section of the nickel-titanium wire and the second section of the nickel-titanium wire, a push cable is obtained. The elastic energy storage efficiency of the first section of the nickel-titanium wire subjected to heat treatment is 32%, the elastic energy storage efficiency of the second section of the nickel-titanium wire subjected to heat treatment is 21%, and the elastic energy storage efficiency of the third section of the nickel-titanium wire not subjected to heat treatment is still 65%.

After the ventricular septum of the heart of a healthy pig is punctured by a puncture system, the push cable is used in a delivery system, and the end subjected to heat treatment is used as a distal end of the push cable. A ventricular septal defect occluder is delivered to a ventricular septal defect of the heart of the pig. The push and release process is smooth, and after release, the distal end of the push cable is not restored to an initial form without obvious damage to heart tissue.

Example 6

A solid nickel-titanium wire is provided, with a wire diameter of 0.5 mm, a length of 1100 mm, and an elastic energy storage efficiency of 80%. The nickel-titanium wire is marked so as to sequentially mark a first section of the nickel-titanium wire, a second section of the nickel-titanium wire, and a third section of the nickel-titanium wire on the nickel-titanium wire. Both ends of the second section of the nickel-titanium wire are connected to the first section of the nickel-titanium wire and the third section of the nickel-titanium wire respectively. The first section of the nickel-titanium wire has a length of 30 mm, the second section of the nickel-titanium wire has a length of 70 mm, and the third section of the nickel-titanium wire has a length of 1000 mm. The first section of the nickel-titanium wire and the second section of the nickel-titanium wire are subjected to heat treatment respectively, and the third section of the nickel-titanium wire is not subjected to heat treatment. The heat treatment of the first section of the nickel-titanium wire includes: placing the first section of the nickel-titanium wire in a heat treatment furnace at a temperature of 360°C, keeping for 50 minutes, cooling to 100°C within 200 minutes, and then naturally cooling to room temperature. The heat treatment of the second section of the nickel-titanium wire includes: placing the second section of the nickel-titanium wire in a heat treatment furnace at a temperature of 400°C, keeping for 40 minutes, cooling to 180°C within 100 minutes, and then naturally cooling to room temperature. After heat treatment of the first section of the nickel-titanium wire and the second section of the nickel-titanium wire, a push cable is obtained. The elastic energy storage efficiency of the first section of the nickel-titanium wire subjected to heat treatment is 27%, the elastic energy storage efficiency of the second section of the nickel-titanium wire subjected to heat treatment is 16%, and the elastic energy storage efficiency of the third section of the nickel-titanium wire not subjected to heat treatment is still 80%.

After the ventricular septum of the heart of a healthy pig is punctured by a puncture system, the push cable is used in a delivery system, and the end subjected to heat treatment is used as a distal end of the push cable. A ventricular septal defect occluder is delivered to a ventricular septal defect of the heart of the pig. The push and release process is smooth, and after release, the distal end of the push cable is not restored to an initial form without obvious damage to heart tissue.

Example 7

Six solid nickel-titanium wires are provided, each with a wire diameter of 0.35 mm, a length of 1450 mm, and an elastic energy storage efficiency of 65%. One end of the nickel-titanium cable (having a length of 90 mm) is placed in a heat treatment furnace at a temperature of 360°C, and the remaining nickel-titanium cable (having a length of 1360 mm) is placed outside the heat treatment furnace. After 60 minutes, the nickel-titanium wire is cooled to 200°C within 120 minutes, and then naturally cooled to room temperature to obtain a push cable. The elastic energy storage efficiency of the end subjected to heat treatment is 21%, and the elastic energy storage efficiency of the end not subjected to heat treatment is still 65%.

After the ventricular septum of the heart of a healthy pig is punctured by a puncture system, the push cable is used in a delivery system, and the end subjected to heat treatment is used as a distal end of the push cable. A ventricular septal defect occluder is delivered to a ventricular septal defect of the heart of the pig. The push and release process is smooth, and after release, the distal end of the push cable is not restored to an initial form without obvious damage to heart tissue.

Example 8

A solid nickel-titanium-copper triple alloy wire (hereinafter referred to as a nickel-titanium-copper wire) is provided, with a wire diameter of 0.6 mm, a length of 1200 mm, and an elastic energy storage efficiency of 75%. One end of the nickel-titanium-copper wire (having a length of 110 mm) is placed in a heat treatment furnace at a temperature of 410°C, and the remaining nickel-titanium-copper wire (having a length of 1100 mm) is placed outside the heat treatment furnace. After 30 minutes, the nickel-titanium wire is cooled to 200°C within 90 minutes, and then naturally cooled to room temperature to obtain a push cable. The elastic energy storage efficiency of the end subjected to heat treatment is 28%, and the elastic energy storage efficiency of the end not subjected to heat treatment is still 75%.

After the ventricular septum of the heart of a healthy pig is punctured by a puncture system, the push cable is used in a delivery system, and the end subjected to heat treatment is used as a distal end of the push cable. A ventricular septal defect occluder is delivered to a ventricular septal defect of the heart of the pig. The push and release process is smooth, and after release, the distal end of the push cable is not restored to an initial form without obvious damage to heart tissue.

Example 9

A solid nickel-titanium-copper wire is provided, with a wire diameter of 0.8 mm, a length of 90 mm, and an elastic energy storage efficiency of 72%. The entire nickel-titanium-copper wire is placed in a heat treatment furnace at a temperature of 410°C, kept for 30 minutes, cooled to 200°C within 90 minutes, and then naturally cooled to room temperature, so as to obtain a nickel-titanium-copper wire with an elastic energy storage efficiency of 27%.

A solid nickel-titanium wire is provided, with a wire diameter of 0.8 mm, a length of 1000 mm, and an elastic energy storage efficiency of 70%. The entire nickel-titanium wire is placed in a heat treatment furnace at a temperature of 430°C, kept for 5 minutes, cooled to 200°C within 50 minutes, and then naturally cooled to room temperature, so as to obtain a nickel-titanium wire with an elastic energy storage efficiency of 47%.

One end of the nickel-titanium-copper wire with the elastic energy storage efficiency of 27% and one end of the nickel-titanium wire with the elastic energy storage efficiency of 47% are fixedly connected by laser welding to obtain a push cable.

After the ventricular septum of the heart of a healthy pig is punctured by a puncture system, the push cable is used in a delivery system, and the end subjected to heat treatment is used as a distal end of the push cable. A ventricular septal defect occluder is delivered to a ventricular septal defect of the heart of the pig. The push and release process is smooth, and after release, the distal end of the push cable is not restored to an initial form without obvious damage to heart tissue.

The various technical features of the above embodiments may be combined randomly. In order to simplify the description, all possible combinations of the various technical features in the above-mentioned embodiments are not described. However, as long as the combinations of these technical features do not contradict, the combinations should be considered to be the scope of the description.

The above-mentioned embodiments express only a few implementations of the present disclosure, which are described in greater detail but are not to be construed as limiting the scope of the present disclosure. It will be appreciated by those of ordinary skill in the art that numerous variations and modifications may be made to the present disclosure without departing from the concept of the present disclosure, which fall within the protection scope of the present disclosure. Therefore, the protection scope of the present disclosure should be determined by the appended claims.

## Claims

1. A push cable, comprising a first cable body and a second cable body, wherein a proximal end of the first cable body is connected to a distal end of the second cable body; the first cable body comprises one or more first metal wires, and the second cable body comprises one or more second metal wires; the one or more first metal wire and the one or more second metal wire are each made of a shape memory alloy material; the elastic energy storage efficiency of the first metal wire is less than an elastic energy storage efficiency of the second metal wire; and in the environment of a human body temperature, when the first cable body is experiencing bending deformation from an initial form, the first cable body is not restored to the initial form without the assistance of an external force.

2. The push cable according to claim 1, wherein the ratio of the elastic energy storage efficiency of the first metal wire to the elastic energy storage efficiency of the second metal wire is in a range of 1:1.5 to 1:5, and the length ratio of the first cable body to the second cable body is in a range of 1:2 to 1:20.

3. The push cable according to claim 1, further comprising a third cable body made of a shape memory alloy material, wherein a distal end of the first cable body is connected to a proximal end of the third cable body.

4. The push cable according to claim 3, wherein the third cable body comprises one or more third metal wires, and an elastic energy storage efficiency of the third metal wire is less than the elastic energy storage efficiency of the second metal wire and greater than the elastic energy storage efficiency of the first metal wire.

5. The push cable according to claim 4, wherein the ratio of the elastic energy storage efficiency of the first metal wire to the elastic energy storage efficiency of the second metal wire is in a range of 1:3 to 1:5, and the ratio of the elastic energy storage efficiency of the third metal wire to the elastic energy storage efficiency of the second metal wire is in a range of 1:1.5 to 1:3.

6. The push cable according to claim 3, wherein the ratio of a total length of the first cable body and the third cable body to a length of the second cable body is in a range of 1:2 to 1:20, and the length ratio of the third cable body to the first cable body is in a range of 1:1 to 1:5.5.

7. The push cable according to claim 1, wherein the second cable body is further provided with a push assistance portion.

8. The push cable according to claim 7, wherein the push assistance portion comprises a braid layer having a concave-convex outer surface.

9. The push cable according to claim 7, wherein the push assistance portion comprises a plurality of abutting members spaced apart along a length direction of the second metal wire.

10. The push cable according to claim 7, wherein the abutting member comprises a sleeve surrounding the second metal wire; or, the abutting member comprises a groove structure.

11. The push cable according to claim 8, wherein the braid layer is further externally provided with a film layer, the film layer wraps the braid layer, and the film layer is formed with a concave-convex outer surface by the braid layer.

12. The push cable according to claim 1, wherein the first cable body sequentially comprises a first sub-section and a second sub-section connected to the first sub-section from the distal end to the proximal end; a distal end of the second sub-section is connected to the first sub-section, and a proximal end of the second sub-section is connected to the second cable body; and the radial dimension of the distal end of the second sub-section is less than the radial dimension of the proximal end of the second sub-section.

13. The push cable according to claim 11, wherein the braid layer comprises a spring tube surrounding the second cable body, the length of the film layer is greater than that of the spring tube, and a distal end of the film layer extends beyond a distal end of the spring tube.

14. The push cable according to claim 13, wherein the distal end of the film layer tightly wraps an outer surface of the second cable body.

**15.** A push system, comprising a handle, a connector, and the push cable according to any one of claims 1 to 14, wherein the distal end of the first cable body is connected to the connector, and a proximal end of the second cable body is connected to the handle.

**16.** A heat treatment method, comprising:

providing a to-be-treated metal member, wherein the to-be-treated metal member is made of a shape memory alloy material, and the elastic energy storage efficiency of the to-be-treated metal member is $\eta_1$; and

placing the to-be-treated metal member in a heat treatment environment of 360°C to 430°C for 5 to 60 minutes, and then cooling the to-be-treated metal member to be less than or equal to 200°C within 50 to 120 minutes to obtain a treated metal member,

wherein the elastic energy storage efficiency of the treated metal member is $\eta_2$, $\eta_1$ and $\eta_2$ are both greater than 0, and $\eta_2$ is less than $\eta_1$.

**17.** A push cable preparation method, comprising:

providing a to-be-treated cable body; and performing heat treatment on one or more sections of the to-be-treated cable body based on the heat treatment method according to claim 16 to obtain a push cable, wherein the push cable comprises a first cable body and a second cable body connected to one end of the first cable body; the first cable body comprises one or more first metal wires, and

the second cable body comprises one or more second metal wires; the elastic energy storage efficiency of the first metal wire is less than the elastic energy storage efficiency of the second metal wire; and in the environment of a human body temperature, when the first cable body is experiencing bending deformation from an initial form, the first cable body is not restored to the initial form without the assistance of an external force.

**18.** A push cable preparation method, comprising:

preparing a first cable body and a second cable body, wherein the first cable body comprises one or more first metal wires, and the second cable body comprises one or more second metal wires; the elastic energy storage efficiency of the first metal wire is less than the elastic energy storage efficiency of the second metal wire; and in the environment of a human body temperature, when the first cable body is experiencing bending deformation from an initial form, the first cable body is not restored to the initial form without the assistance of an external force; and fixedly connecting a proximal end of the first cable body to a distal end of the second cable body to obtain a push cable.

100

20

30

11 12

10

**FIG. 1**

40

B

D

C

42

β

α

41

A

**FIG. 2**

**FIG. 3**

**FIG. 4**

**FIG. 5**

**FIG. 6**

**FIG. 7**

**FIG. 8**

FIG. 9

FIG. 10

S01: Provide a to-be-treated metal member

S02: Place the metal member in a heat treatment environment of 360°C to 430°C for 5 to 60 minutes

S03: Cool the metal member to be less than or equal to 200°C in the heat treatment environment for 50 to 120 minutes to obtain a treated metal member

**FIG. 11**

S10: Provide a to-be-treated cable body

S20: Perform heat treatment on one or more sections of the to-be-treated cable body

**FIG. 12**

S100: Prepare a first cable body and a second cable body respectively

S200: Fixedly connect one end of the first cable body to one end of the second cable body

**FIG. 13**

**FIG. 14**

**FIG. 15**

**FIG. 16**

## INTERNATIONAL SEARCH REPORT

| International application No. |
| --- |
| **PCT/CN2022/133472** |

**A. CLASSIFICATION OF SUBJECT MATTER**

A61B17/00(2006.01)i

According to International Patent Classification (IPC) or to both national classification and IPC

**B. FIELDS SEARCHED**

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNABS, CNTXT, CNKI, VEN, WPABS, WPABSC, ENTXT, ENTXTC: 推送, 缆, 弹性储能, 应力, 应变, 体温, 形状记忆, 钛镍, 镍钛, 能量密度, push+, cable?, elastic w energy, storage, stress, strain, temperture, shape w memory, Ti, Ni, energy w density

**C. DOCUMENTS CONSIDERED TO BE RELEVANT**

| Category* | Citation of document, with indication, where appropriate, of the relevant passages | Relevant to claim No. |
| --- | --- | --- |
| X | US 6068623 A (PERCUSURGE INC.) 30 May 2000 (2000-05-30) description, columns 7-22, and figures 1-9c | 1-18 |
| A | CN 109984779 A (LIFETECH SCIENTIFIC (SHENZHEN) CO., LTD.) 09 July 2019 (2019-07-09) entire document | 1-18 |
| A | CN 112971872 A (LIFETECH SCIENTIFIC (SHENZHEN) CO., LTD.) 18 June 2021 (2021-06-18) entire document | 1-18 |
| A | CN 107011647 A (SICHUAN UNIVERSITY) 04 August 2017 (2017-08-04) entire document | 1-18 |
| A | CN 111388143 A (ZHAO XIAOHUI) 10 July 2020 (2020-07-10) entire document | 1-18 |
| A | CN 111518377 A (SICHUAN UNIVERSITY) 11 August 2020 (2020-08-11) entire document | 1-18 |

☑ Further documents are listed in the continuation of Box C.     ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **16 February 2023** | **20 February 2023** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

| International application No. |
| --- |
| **PCT/CN2022/133472** |

### C. DOCUMENTS CONSIDERED TO BE RELEVANT

| Category* | Citation of document, with indication, where appropriate, of the relevant passages | Relevant to claim No. |
| --- | --- | --- |
| A | US 2006074403 A1 (RAFIEE NASSER) 06 April 2006 (2006-04-06)<br>entire document | 1-18 |
| A | US 2011251364 A1 (ANTHAMATTEN MITCHELL L; LI JIAHUI;) 13 October 2011 (2011-10-13)<br>entire document | 1-18 |

Form PCT/ISA/210 (second sheet) (July 2022)

**INTERNATIONAL SEARCH REPORT**
Information on patent family members

International application No.

**PCT/CN2022/133472**

| Patent document cited in search report | | | Publication date (day/month/year) | Patent family member(s) | | | Publication date (day/month/year) |
|---|---|---|---|---|---|---|---|
| US | 6068623 | A | 30 May 2000 | JP | 2001514544 | A | 11 September 2001 |
| | | | | AU | 6691698 | A | 22 September 1998 |
| | | | | US | 6217567 | B1 | 17 April 2001 |
| | | | | US | 6375628 | B1 | 23 April 2002 |
| | | | | EP | 0996480 | A2 | 03 May 2000 |
| | | | | CA | 2287072 | A1 | 11 September 1998 |
| | | | | US | 2002095137 | A1 | 18 July 2002 |
| | | | | WO | 9839048 | A2 | 11 September 1998 |
| CN | 109984779 | A | 09 July 2019 | None | | | |
| CN | 112971872 | A | 18 June 2021 | None | | | |
| CN | 107011647 | A | 04 August 2017 | None | | | |
| CN | 111388143 | A | 10 July 2020 | None | | | |
| CN | 111518377 | A | 11 August 2020 | None | | | |
| US | 2006074403 | A1 | 06 April 2006 | None | | | |
| US | 2011251364 | A1 | 13 October 2011 | US | 8172873 | B2 | 08 May 2012 |

Form PCT/ISA/210 (patent family annex) (July 2022)